# EUROPEAN PATENT APPLICATION

(11) **EP 4 578 418 A1**
(43) Date of publication of application: **02.07.2025**
(21) Application number: 23315495.4
(22) Date of filing: 28.12.2023
(51) Int. Cl.: A61B 34/10, A61B 34/00

(54) **SYSTEM, METHOD, COMPUTER PROGRAM AND COMPUTER PROGRAM PRODUCT FOR AUTOMATIC PLANNING OF A CLINICAL INTERVENTION FOR IMPLANTING A CARDIAC VALVE**

(71) Applicant: Caranx Medical, 75013 Paris (FR)
(72) Inventor: BERTHET-RAYNE Pierre, 06800 Cagnes-sur-Mer (FR); OURAHLI, Sarah, 06000 Nice (FR); CERRUTI, Giulio, 06700 Saint-Laurent-Du-Var (FR); SMITS, Jonas Victor Harmen, 3360 Bierbeek (BE); MIRA, Anna, 06000 Nice (FR); PRZYBYLSKI, Flavie, 06800 Cagnes sur Mer (FR); MOSCU, Mircea, 06600 Antibes (FR); WEI, Wen, 06410 Biot (FR); WAUTERS, Loic, 06200 Nice (FR); BENOIST, Camille, 06570 Saint Paul de Vence (FR); LITTLE, Kieran, 06000 Nice (FR); SCHEGG, Pierre, 06000 Nice (FR); AGNUS, Vincent, 67400 Illkirch-Graffenstaden (FR)
(74) Representative: Hepp Wenger Ryffel AG

(57) **Abstract**

The present invention relates to a system, a method, a computer program and a computer program product for automatic planning and/or preparing of a clinical intervention for implanting a cardiac valve in a patient. The system (100) comprises an input interface (101) for entering data and a computing unit (102). Said computing unit (102) is adapted to receive input data via the input interface (101). The input data comprise patient data (201) relating to characteristics of the patient, cardiac valve data (202) relating to the orientation of a prosthetic cardiac valve (202) with respect to a delivery tool (401) to be used for implantation and/or with respect to a loading tool (402) for loading the prosthetic cardiac valve (202) into the delivery tool (401), preferably, placement data (203) relating to the placement of the delivery tool (401) with respect to the patient, in particular before insertion of the delivery tool (401) into the vessel of the patient. The computing unit (102) is adapted to determine orientation data (300) relating to the expected orientation of the prosthetic cardiac valve (2) with respect to the native cardiac valve (1), in particular to the position of the coronaries (RCA, LCA) and/or to the position commissures (4), in the final position of the prosthetic cardiac valve (1) on basis of the input data.

## Description

The present invention relates to a system, a method, a computer program and a computer program product for automatic planning and/or preparing of a clinical intervention for implanting a cardiac valve in a patient according to the independent claims.

Implanting a prosthetic cardiac valve may concern an aortic valve replacement, a mitral replacement, a tricuspid valve replacement or a pulmonary valve replacement by an artificial heart valve. Current aortic valve replacement approaches may include closed heart surgery. Trans-catheter aortic valve implantation (TAVI) or trans-catheter aortic valve replacement (TAVR) is a minimally invasive procedure for treating a failing aortic valve.

A valve implant (e.g. a bioprosthetic valve made of e.g. porcine or bovine pericardium sutured on a metal stent) typically is crimped inside a catheter. The catheter is inserted, for example, via the femoral artery and is pushed upstream along the aorta up to the diseased native valve, within which the valve implant is deployed. Main complications during implantation are vascular injury, stroke, cardiac injury, aortic regurgitation, cardiac conduction abnormalities and valve misplacement.

In particular, the orientation of the implanted prosthetic cardiac valve should comply with the orientation of the commissures of the native valve to provide an optimal access to the coronaries after the implantation.

Due to a commissural misalignment, the coronary access may be limited, and consequently the coronary artery flow may be disturbed. There may arise a stress and a strain to the leaflets, and in the worst case a central leak. The effectiveness of an outer sealing skirt may be affected due to misalignment. The grade of commissural alignment or misalignment can be detected by a fluoroscopic image. In case of misalignment, it might be necessary to reposition or even to reimplant the valve.

Typically, the implantation process is image guided and makes use of patient images taken and annotated in the planning stage. Identifying how to perform the procedure optimally is time consuming, requires considerable skills of the operator and bears the risk of errors.

Co-pending European application EP23315250.3 discloses a system and a method for automatic planning of a clinical intervention for implanting a cardiac valve in a patient, wherein a selected surgical tool and/or a selected surgical method are selected on basis of input data. The input data may comprise characteristics relevant for commissural alignment, which may relate to the intended orientation of an implant valve at the implant position and/or to an orientation of an implant valve within a delivery device, for example to the valve crimping position, which may be adjustable and/or which has to be taken into account.

Preparing of a clinical intervention for implanting a prosthetic cardiac valve implant may include loading a prosthetic valve into a delivery tool, in particular using a loading tool, such as a crimping device.

It is an object of the present invention to overcome the drawbacks of the prior art. In particular, the system and the method according to the present invention shall provide proper commissural alignment..

These and other objects are solved with a system, a method and a computer program according to the independent claims.

The system for an at least partially automatic planning and/or preparing of a clinical intervention for implanting a prosthetic cardiac valve in a patient comprises an input interface for entering data and a computing unit.

Said computing unit is adapted to receive input data via the input interface.

The input interface preferably is selected from the group of a keyboard, a vocal interface, a mechanical button or switch, a mouse, joystick, haptic glove, a graphical user interface, in particular a touchscreen, a motion detector, in particular a gaze tracker or a head motion detector, a virtual reality or an augmented reality interface, and a 3D monitor, in particular a hologram presentation combined with a haptic glove or a motion detection.

The input data comprise patient data relating to characteristics of the patient.

The patient data relating to characteristics of the patient may comprise imaging data, in particular 2D and/or 3D imaging data and/or 2D sliced imaging data.

The 3D imaging data may include one or a combination of MRI (Magnetic resonance imaging)-data, CBCT (Cone-beam computed tomography)-data, multi-view ultrasound data, 3D ultrasound data and data of a surface scanning, for example using pressure sensors or a balloon.

The patient data relating to characteristics of the patient may relate to (i) the vessel shape, in particular of vessels on the path from the access point to the native valve, such as the femoral artery and the aorta, such as vessel length, width and tortuosity, and/or may relate to (ii) the vessel state, such as the presence of an aneurism and/or a thrombosis, the existence of calcifications and/or plaques.

The patient data relating to characteristics of the patient may relate to characteristics of the native valve to be functionally replaced, for example shape, such as diameter, and state of native valve, such as kind of malfunction or grade of calcification and orientation of the commissures and the shape of the left ventricular outflow tract.

The patient data relating to characteristics of the patient may comprise information about an insertion tool, in particular regarding type and size, and when already fixed to the patient and the axis of the insertion tool coincides with the axis of the vessel relating to the rotational orientation of the insertion tool with respect to the vessel axis.

The input data further comprise cardiac valve data relating to the orientation of a prosthetic cardiac valve with respect to a delivery tool to be used for implantation and/or with respect to a loading tool for loading the prosthetic cardiac valve into the delivery tool.

Within this context the terms "orientation" and "rotation" relate to the valve axis as a rotation axis. Typically, when arranged in the delivery tool or in the loading tool, the prosthetic valve is concentrically arranged in at least a part of the tool, such that as the valve axis coincides with the axis of the respective part of the tool. Even when the tool axis and the valve axis coincide, also when the prosthetic valve is not arranged within the tool, the valve may have different orientations within respect to the respective tool as there is still a rotational degree of freedom. The orientation may be given by a rotational angle between a specific mark on the prosthetic valve and a specific mark on the tool.

Cardiac valve data relating to the orientation of a prosthetic cardiac valve with respect to a delivery tool to be used for implantation may include information about the orientation of the prosthetic cardiac valve with respect to a typical part of the delivery device, for example an introducer sheath and/or to a delivery balloon.

The input data may further comprise placement data relating to the placement of the loading tool with respect to the delivery tool, in particular before loading the prosthetic cardiac valve into the delivery tool.

The orientation of the loading tool with respect to the delivery tool may be predetermined in order to provide an unambiguous connection between the loading tool and the delivery tool. Alternatively, there may a rotational degree of freedom, when functionally connecting the loading tool and the delivery tool.

A combination of placement data relating to the placement of the loading tool with respect to the delivery tool and the orientation of a prosthetic cardiac valve with respect to the loading tool may result in the orientation of a prosthetic cardiac valve with respect to a delivery tool.

The input data may further comprise placement data relating to the placement of the delivery tool with respect to the patient, in particular before insertion of the delivery tool into the vessel of the patient.

The orientation of the delivery tool with respect to an insertion tool, which may be fixed to the patient, may be predetermined in order to provide an unambiguous access of the delivery tool into the insertion tool. Alternatively, there may a rotational degree of freedom, when inserting the delivery tool into the insertion tool.

Placement data relating to the placement of the delivery tool with respect to the patient may include orientation data relating to the rotational orientation of the delivery tool with respect to a vessel axis when the axis of the valve holding part of the delivery tool coincides with the axis of the vessel, relating to the rotational orientation of the delivery tool with respect to an the axis of an insertion tool when the axis of the valve holding part coincides with the axis of the insertion tool.

Placement data may also include an angle between the axis of the valve holding part and the axis of the vessel and/or position data of the delivery tool with respect to a reference system such as the room.

Said computing unit is further adapted to determine orientation data relating to the expected orientation of the prosthetic cardiac valve with respect to the native cardiac valve. The orientation with respect to the native cardiac valve may be the orientation with respect to any native valve feature, but also an orientation with respect to features of the environment of the native valve. )In particular, the expected orientation may be an orientation with respect to the position of at least one the coronaries and/or to the position of at least on commissure of the native valve, in the final position of the prosthetic cardiac valve on basis of the input data.

Orientation data relating to the expected orientation of the prosthetic cardiac valve may for example be given by an angle between the commissures of the native valve and the commissures of the prosthetic valve.

On basis of an initial orientation of the valve, in particular before insertion into the patient, of the characteristics of the used tools and of the characteristics of the patient, the system may determine the final orientation of the prosthetic valve.

Thus, before delivery a user is able to anticipate the final position of the prosthetic valve.

This determination of orientation data provides the possibility to improve the delivery process of the prosthetic valve and the preparation of the delivery in order to achieve an optimized orientation of the deployed prosthetic valve.

The computing interface may be adapted to determine orientation data on basis of input data, received before and/or during the delivery procedure. Thus, the computing interface may be adapted to determine and/or to update orientation data also during the delivery process.

The input data may comprise data relating to the type and/or size of the prosthetic cardiac valve. The input data may also comprise data relating to local hemodynamics such as pressure and/or flow gradient.

Data relating to the type of the cardiac valve may relate to the shape of the valve to be implanted and the target position and/or the intended target orientation of the valve to be implanted with respect to the native valve to be functionally replaced, in particular the intended implantation depth, the tilt and the orientation of the valve to be implanted.

In particular, data relating to the type of the prosthetic cardiac valve may comprise information about a specific feature which can be used as a reference mark for characterizing the rotational orientation of the prosthetic valve with respect to the delivery tool, the loading tool, a vessel of the patient and/or the native valve.

The computing unit may be adapted to provide output data.

The output data may comprise output data for displaying orientation data on an output device.

The output data may comprise output data related to an optimized orientation of the prosthetic cardiac valve with respect to the delivery tool, in particular for providing signals and/or instructions to rotate the prosthetic cardiac valve within the delivery tool and/or for providing signals and/or instructions for loading the prosthetic cardiac valve into the delivery tool.

An optimized orientation of the prosthetic cardiac valve with respect to the delivery tool results in an expected orientation of the prosthetic cardiac valve with respect to the native cardiac valve wherein the prosthetic valve is at least not completely misaligned.

Signals and/or instructions may provide for directly and automatically controlling a respective tool.

Signals and/or instructions may inform a user to control a respective tool.

The output data may comprise output data related to an optimized position of the delivery tool with respect to the patient before insertion of the delivery tool into the vessel of the patient, in particular for providing signals and/or instructions to change the position of the delivery tool with respect to patient, preferably for providing signals and/or instructions to change the position of the delivery tool with respect to an insertion device, and/or for providing signals and/or instructions to change the position of the insertion device with respect to the patient.

Data related to an optimized position of the delivery tool with respect to the patient may include information about the angles for insertion the delivery device into the vessel and to orientate the cardiac valve in a proper way, that is to choose the proper angle between vessel axis and valve axis, and to choose a rotation angle of the valve with respect to vessel and/or valve axis.

The output data may comprise output data related to an optimized navigation of the delivery tool to the final position of the cardiac valve, in particular for providing navigation data in terms of movement instructions. Movement instructions may include requests to rotate the delivery tool in certain direction when achieving at a certain position.

The output data may comprise output data related to a risk value based on the orientation data.

The risk value may be related to the orientation data and as the orientation data may be updated during the delivery process, also the risk value may be updated.

The computing unit may be adapted to provide output data for carrying out a procedure selected from loading the prosthetic valve or a valve component into the delivery tool, moving and in particular rotating the prosthetic valve with respect to a loading tool, moving and in particular rotating the prosthetic cardiac valve within the delivery tool, positioning the delivery tool with respect to the patient, navigating the delivery tool to the vicinity of the final position.

Output data concerning the loading the prosthetic valve into the delivery tool may comprise data being related to the orientation of the prosthetic valve with respect to the delivery tool and/or to the orientation of a loading tool with respect to the delivery tool.

Output data concerning the rotating the prosthetic valve with respect to a loading tool may be related to a rotation of the valve before, during and/or after being crimping.

Output data concerning the positioning of the delivery tool with respect to the patient may relate to the positioning of the delivery tool with respect to an insertion tool.

The cardiac valve data may comprise an annular position of a characteristic feature of the prosthetic cardiac valve placed in a delivery device with respect to the delivery device.

The cardiac valve data may comprise an annular position of characteristic feature of the prosthetic cardiac valve with respect to a loading tool for loading the prosthetic cardiac valve to the delivery device, in particular a crimping device.

The characteristic feature of the prosthetic cardiac valve may be a suture hole, a commissural post, a fixation element and/or a marker, for example attached to a metal support.

The computing unit may be adapted to create a virtual model representing a body part involved in the procedure and the prosthetic valve to represent the orientation data. The computing unit may be adapted to simulate the navigation of the prosthetic valve through the delivery path and/or to determine suitable positions for rotation and/or rotation angles of the delivery tool during delivery.

The computing unit may be adapted for providing output data to an output interface, in particular the system may comprise an output interface.

The output interface may comprise an output device for representing output data, such as a monitor, in particular a 3D monitor, a display, a virtual reality or an augmented reality interface, a warning indicator and/or a visual pointer or an acoustic output such as a synthetic voice generator. The synthetic voice may be generated in any desired language.

The visual pointer may be a laser pointer, preferably to be arranged in a predetermined relationship with respect to the patient or to a representation of the patient. The laser pointer may highlight a path and/or a specific position, for example on a 2D or 3D representation of a virtual model of a body part.

The output interface may be connected or connectable to the delivery tool, to an insertion tool and/or to a loading tool, such as a crimping device. The output interface may provide control commands to the delivery tool, the insertion tool and/or the loading tool and/or may provide output data to an output device of the delivery tool, the insertion tool and/or the loading tool.

An assembly may comprise a system as described above and a at least one of a delivery tool, an insertion tool, a loading tool, a display device, an imaging device and a prosthetic valve or valve component.

The delivery tool and/or the loading tool may comprise means for rotating the prosthetic cardiac valve, for example elements, which allow active rotation and/or passive rotation and/or selforientation of the prosthetic valve around the valve axis.

A rotation controller may be integrated in the delivery system, which allows for simple and straightforward alignment.

An imaging device, such as an X-ray device, an MRT (magnetic resonance tomography) device, an MDCT (multi detector computed tomography) device and/or a fluoroscopy device, may provide data for representing at least a part of the patient's body.

A method for computer based automatic planning and/or preparing of a clinical intervention for implanting a cardiac valve in a patient may use a system as described above.

The method comprises the following steps.

Input data relating to characteristics of the patient are received via an input interface.

Input data are received relating to the orientation of a cardiac valve to be implanted with respect to a delivery tool to be used for implantation and/or with respect to a loading tool for loading the prosthetic cardiac valve into the delivery tool.

Preferably, input data relating to the placement of the delivery tool with respect to the patient are received, in particular before insertion of the delivery tool into the vessel of the patient.

Input data are received via an input interface. Input data correspond to input data as described above.

The method comprises the step of determining orientation data relating to the orientation of the cardiac valve with respect to the native valve or a feature of a valve environment, in particular to the position of at least one coronary and/or to the position of at least one commissure, in the final position.

Orientation data correspond to orientation data as described above.

The method may comprise the further step of receiving data relating to the type and/or size of the cardiac valve or to local hemodynamics such as pressure and/or flow gradient. Data relating to the type and/or size of the cardiac valve correspond to cardiac valve data as described above.

The method may comprise the further step of providing output data as described above.

The output data may be selected from output data for displaying orientation data on an output device, output data related to an optimized orientation of the prosthetic cardiac valve with respect to the delivery tool, in particular providing signals and/or instructions to move and in particular to rotate the prosthetic cardiac valve within delivery tool and/or for providing signals and/or instructions for loading into the delivery tool, output data related to an optimized position of the delivery tool with respect to the patient before insertion of the delivery tool into the vessel of the patient, in particular for providing signals and/or instructions to change the position of the delivery tool with respect to patient, preferably providing signals and/or instructions to change the position of the delivery tool with respect to an insertion device, output data related to an optimized navigation of the delivery tool to the final position of the cardiac valve, in particular providing navigation data in terms of movement instructions and output data related a to risk value based on the orientation data.

The method may comprise the further step of carrying out a procedure selected from loading the prosthetic valve or a valve component into the delivery tool, moving and in particular rotating the prosthetic valve with respect to the loading tool, moving and in particular rotating the prosthetic cardiac valve with respect to the delivery tool, in particular within the delivery tool.

The method may comprise the further step of carrying out a procedure selected from positioning the delivery tool with respect to the patient, in particular positioning the delivery tool with respect to an insertion tool and navigating the delivery tool to the final position. Additionally, or alternatively, the method may comprise the steps of interrupting or stopping the valve delivery and/or retrieving the valve or a valve component. This may be beneficial in particular if during the procedure, problems or risks become apparent.

The method may comprise the step of receiving cardiac valve data, wherein the cardiac valve data is selected from an annular position of a characteristic feature of the prosthetic cardiac valve placed in a delivery device with respect to the delivery device, and an annular position of characteristic feature of the prosthetic cardiac valve with respect to a loading device for loading the prosthetic cardiac valve to the delivery device, in particular a crimping device.

The method may comprise the further step of creating a virtual model representing a body part involved in the procedure and the prosthetic valve to represent the orientation data.

A computer program according to the invention comprises a program code for carrying out the steps of the method as described above when the program is executed on a computer, in particular on a computer of a system as described above and/or an assembly as described above.

A computer program product according to the invention can be loaded directly into an internal memory of a digital computer and comprises software code portions executing the method as described above when the program is running on the digital computer of a system as described above and/or an assembly as described above.

The invention is explained in the following by means of exemplary embodiments and the accompanying drawing, in which:
- Fig. 1: is a schematic presentation of a system according to the invention;
- Fig. 2: shows a view to the annular plane of an aortic root having (i) a native valve and (ii) having an implanted valve.

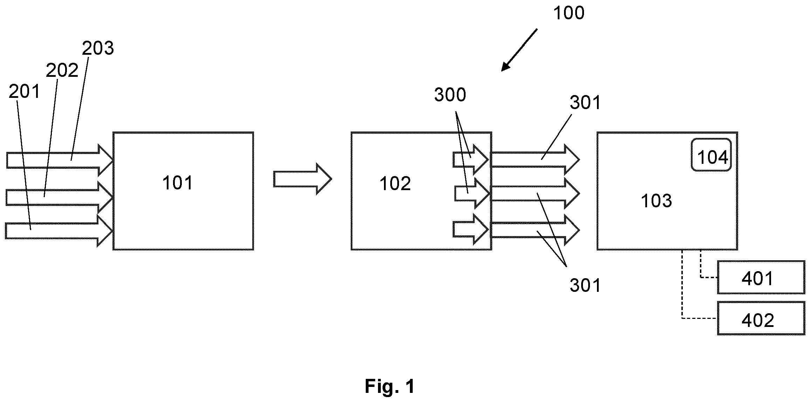

Fig. 1 is a schematic presentation of a system 100 according to the invention.

The system 100 is for automatic planning and/or preparing of a clinical intervention for implanting a prosthetic cardiac valve in a patient.

The system 100 comprises an input interface 101 for entering data, a computing unit 102 and an output interface 103.

The computing unit 102 is adapted to receive input data 201, 202, 203 via the input interface 101.

The input data 201, 202, 203 comprise patient data 201 relating to characteristics of the patient, cardiac valve data 202 relating to the orientation of a prosthetic cardiac valve with respect to a delivery tool to be used for implantation and/or with respect to a loading tool for loading the prosthetic cardiac valve into the delivery tool and placement data 203 relating to the placement of the delivery tool with respect to the patient.

The computing unit 102 is adapted to determine orientation data 300 relating to the expected orientation of the prosthetic cardiac valve with respect to the native cardiac valve, in particular to the coronaries position and/or to the commissural position, in the final position of the prosthetic cardiac valve on basis of the input data.

The computing unit 102 is adapted to provide output data 301 on basis of the orientation data 300 relating to the expected orientation of the prosthetic cardiac valve to the output device 103.

The output data 301 is selected from output data 301 for displaying orientation data 300 on an output device 104, output data 301 related to an optimized orientation of the prosthetic cardiac valve with respect to the delivery tool, output data 301 related to an optimized position of the delivery tool with respect to the patient before insertion of the delivery tool into the vessel of the patient, output data 301 related to an optimized navigation of the delivery tool 401 to the final position of the cardiac valve and output data 301 related to a risk value based on the orientation data.

The output data 301 may be displayed on the output device 104.

The output device 103 may use output data 301 for controlling a delivery tool 401 and/or a loading tool 402.

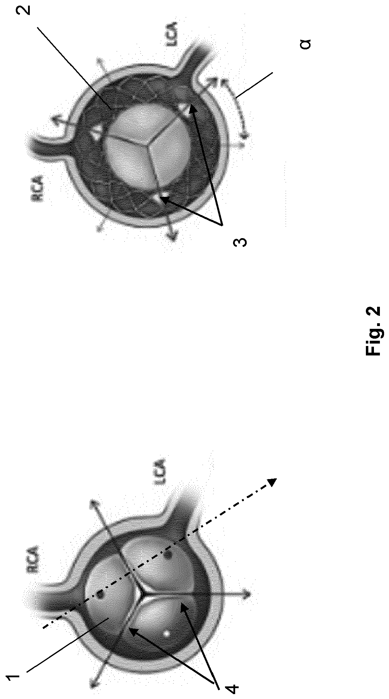

Fig. 2 shows the annular plane of an aortic root heart having (i) a native valve 1 and (ii) having an implanted prosthetic cardiac valve 2.

The commissures 3 of the implanted prosthetic cardiac valve 2 may have an orientation that deviates by an angle α from the orientation of the commissures 4 of the native valve 1.

As long as the deviation angle α is between 0° and 15°, "commissural alignment" is assumed. The path to the right coronary artery RCA and to the left coronary artery LCA remains undisturbed.

A "mild" commissural misalignment is assumed for an angle range between 15° and 30°, a "moderate" commissural misalignment is assumed for an angle range between 30° and 45° and a "severe" commissural misalignment is assumed for an angle range between 45° and 60°.

Due to commissural misalignment the coronary access may be limited, and consequently the coronary artery flow may be disturbed. There may arise a stress and a strain to the leaflets, and in the worst case a central leak. The effectiveness of the outer sealing skirt may be affected due to misalignment, and it might be necessary to reimplant the valve.

In a view, which brings neighbouring cusps to overlap (see dashed arrow), one of the TAVI-valve commissural posts should be lateralized at the right side of the fluoroscopic image. Alternatively, a view which brings the right and the left coronary ostia to overlap may be used.

Orientation data 300 relating to the expected orientation of the prosthetic cardiac valve 2 given by the deviation angle α and defining a grade of commissural alignment or misalignment can be determined by the system 100 on basis of input data relating to the patient, to the delivery tool 401 and to the prosthetic cardiac valve 2.

For automatically determining the grade of commissural alignment of an implant valve, a 3D geometrical model of the, preferably patient specific, aortic anatomy from femoral access to the annular ring, in particular a rigid model, a valve crimping position and a delivery device orientation before implantation can be used as input data.

Optionally, a 3D biomechanical model of the patient artery can be taken into account as further input data, which for example provides the individual eccentricity of the coronaries RCA, LCA with respect to the native valve 1 and which may be provided by a fluoroscopy imaging method.

A model simulating the interaction of the delivery device with the static 3D anatomy or/and a model simulating the interaction of the delivery device with the dynamic 3D anatomy can be provided by the computing unit (see figure 1).

A reduced model of the delivery device can be used as input data.

On basis of the calculated orientation data for example given by the grade of the commissural alignment an optimal valve crimping position and an optimal delivery device orientation before implantation may be provided as output data.

According to the intended final position the computing unit may provide an output on how to crimp the valve on the delivery device and at which angle the delivery device should be inserted into the artery and into the native valve.

## Claims

1. A system for an at least partially automatic planning and/or preparing of a clinical intervention for implanting a prosthetic cardiac valve (2) in a patient, the system (100) comprising
- an input interface (101) for entering data,
- a computing unit (102),
wherein said computing unit (102) is adapted
- to receive input data (201, 202, 203) via the input interface (101), the input data comprising
∘ patient data (201) relating to characteristics of the patient,
∘ cardiac valve data (202) relating to the orientation of a prosthetic cardiac valve (202) with respect to a delivery tool (401) to be used for implantation and/or with respect to a loading tool (402) for loading the prosthetic cardiac valve (202) into the delivery tool (401),
∘ preferably, placement data (203) relating to the placement of the delivery tool (401) with respect to the patient, in particular before insertion of the delivery tool (401) into the vessel of the patient,
- to determine orientation data (300) relating to the expected orientation of the prosthetic cardiac valve, (2) with respect to the native cardiac valve or a feature of a valve environment (1), in particular to the position of the coronaries (RCA, LCA) and/or to the position commissures (4), in the final position of the prosthetic cardiac valve (1) on basis of the input data (201, 202, 203).

2. A system according to claim 1, wherein the input data comprise data relating to at least one of (i) the type of the prosthetic cardiac valve (2), (ii) the size of the prosthetic cardiac valve (2) (iii) local hemodynamics such as pressure and/or flow gradient.

3. A system according to claim 1 or 2, wherein the computing unit is adapted to provide output data (301), wherein the output data (301) is selected from
- output data (301) for displaying orientation data on an output device (104),
- output data (301) related to an optimized orientation of the prosthetic cardiac valve (2) with respect to the delivery tool (401), in particular for providing signals and/or instructions to rotate the prosthetic cardiac valve (2) within the delivery tool (401) and/or for providing signals and/or instructions for loading into the delivery tool (401),
- output data (301) related to an optimized position of the delivery tool (401) with respect to the patient before insertion of the delivery tool (401) into the vessel of the patient, in particular for providing signals and/or instructions to change the position of the delivery tool (401) with respect to patient, preferably for providing signals and/or instructions to change the position of the delivery tool (401) with respect to an insertion device,
- output data (301) related to an optimized navigation of the delivery tool (401) to the final position of the prosthetic cardiac valve (2), in particular for providing navigation data in terms of movement instructions and
- output data (301) related to a risk value based on the orientation data.

4. A system according to one of the preceding claims, wherein' the computing unit is adapted to provide output data (301) for carrying out a procedure selected from
- loading the prosthetic cardiac valve (2) or a valve component into the delivery tool,
- moving, in particular rotating, the prosthetic cardiac valve (2) with respect to a loading tool;
- moving, in particular rotating, the prosthetic cardiac valve (2) within the delivery tool,
- positioning the delivery tool (401) with respect to the patient,
- navigating the delivery tool (401) to the vicinity of the final position.

5. A system according to any of the preceding claims, wherein the prosthetic cardiac valve (2) data may comprise at least one of
- an annular position of a characteristic feature of the prosthetic cardiac valve (2) placed in a delivery device with respect to the delivery device,
and
- an annular position of characteristic feature of the prosthetic cardiac valve (2) with respect to a loading tool (402) for loading the prosthetic cardiac valve to the delivery device, in particular a crimping device.

6. A system according to any of the preceding claims, wherein the computing unit (102) is adapted to create a virtual model representing a body part involved in the procedure and the prosthetic cardiac valve (2) to represent the orientation data.

7. A system according to any of the preceding claims, wherein the computing unit (102) is adapted for providing output data (301) to an output interface (103), in particular the system comprising an output interface (103).

8. A system according to claim 7, wherein the output interface (103) comprises an output device (104) for representing output data (301),
such as
- a monitor, in particular a 3D monitor,
- a display,
- a virtual reality or an augmented reality interface,
- a warning indicator,
- a visual pointer
- an acoustic output such as a synthetic voice generator.

9. A system according to claim 7 or 8, wherein the output interface (103) is connected or connectable to the delivery tool, to an insertion tool and/or to a loading tool.

10. An assembly comprising a system according to any of the preceding claims, and further comprising at least one of
- a delivery tool,
- an insertion tool,
- a loading tool,
- a display device,
- an imaging device,
- a prosthetic valve or valve component.

11. A method for computer based automatic planning and/or preparing of a clinical intervention for implanting a prosthetic cardiac valve (2) in a patient, preferably using a system according to one of claims 1 to 9, the method comprising the steps of
- receiving input data (201) relating to characteristics of the patient via an input interface (101);
- receiving input data (202) relating to the orientation of a prosthetic cardiac valve (2) to be implanted with respect to a delivery tool (401) to be used for implantation, and/or with respect to a loading tool (402) for loading the prosthetic cardiac valve (2) into the delivery tool;
- preferably receiving input data (203) relating to the placement of the delivery tool (401) with respect to the patient, in particular before insertion of the delivery tool (401) into the vessel of the patient;
- determining orientation data (300) relating to the orientation of the prosthetic cardiac valve (2) with respect to the native valve (1) or a feature of a valve environment, in particular to the position of at least one the coronary (RCA, LCA) and/or to the position of at least one commissure (4), in the final position.

12. A method according to claim 11 comprising the further step of receiving data relating to the type and/or size of the prosthetic cardiac valve (2) or to local hemodynamics such as pressure and/or flow gradient.

13. A method according to claim 11 or 12 comprising the further step of providing output data (301), wherein the output data is selected from
- output data (301) for displaying orientation data on an output device (104),
- output data (301) related to an optimized orientation of the prosthetic cardiac valve (2) with respect to the delivery tool, in particular providing signals and/or instructions to move the prosthetic cardiac valve (2) within delivery tool (401) and/or for providing signals and/or instructions for loading into the delivery tool,
- output data (301) related to an optimized position of the delivery tool (401) with respect to the patient before insertion of the delivery tool (401) into the vessel of the patient, in particular for providing signals and/or instructions to change the position of the delivery tool (401) with respect to patient, preferably providing signals and/or instructions to change the position of the delivery tool (401) with respect to an insertion device,
- output data (301) related to an optimized navigation of the delivery tool (401) to the final position of the cardiac valve, in particular providing navigation data in terms of movement instructions,
and
- output data (301) related a to risk value based on the orientation data (300).

14. A method according to any of claims 11 to 13 comprising the further step of carrying out a procedure selected from
- loading the prosthetic cardiac valve or a valve component (2) into the delivery tool;
- moving and in particular rotating the prosthetic cardiac valve (2) with respect to the loading tool;
- moving and in particular rotating the prosthetic cardiac valve (2) with respect to the delivery tool, in particular within the delivery tool.

15. A method according to any of claims 11 to 14 comprising the further step of carrying out a procedure selected from
- positioning the delivery tool (401) with respect to the patient,
- navigating the delivery tool (401) to the final position
- interrupting or stopping the valve delivery
- retrieving the valve or a valve component.

16. A method according to any of claims 11 to 15 comprising the step of receiving cardiac valve data (202), wherein the cardiac valve data (202) is selected from
- an annular position of a characteristic feature of the prosthetic cardiac valve (2) placed in a delivery device with respect to the delivery device,
and
- an annular position of characteristic feature of the prosthetic cardiac valve (2) with respect to a loading device for loading the prosthetic cardiac valve to the delivery device, in particular a crimping device.

17. A method according to any of claims 11 to 16 comprising the further step of creating a virtual model representing a body part involved in the procedure and the prosthetic cardiac valve (2) to represent the orientation data (300).

18. A computer program comprising program code for carrying out the steps of the method according to any one of the claims 11 to 17 when the program is executed on a computer of a system according to one of claims 1-9 and/or an assembly according to claim 10.

19. A computer program product which can be loaded directly into an internal memory of a digital computer and which comprises software code portions executing the method steps of at least one of the claims 11 to 17 when the program is running on the digital computer of a system according to one of claims 1-9 and/or an assembly according to claim 10.
